# EUROPEAN PATENT APPLICATION

(11) **EP 2 106 791 A1**
(43) Date of publication of application: **07.10.2009**
(21) Application number: 08153701.1
(22) Date of filing: 31.03.2008
(51) Int. Cl.: A61K 31/198, A61P 9/00

(54) **Glutamine or glutamine-containing dipeptide in a specific dosage for the treatment of inflammation**

(71) Applicant: Biotempt B.V., 7958 NZ Koekange (NL)
(72) Inventor: The designation of the inventor has not yet been filed
(74) Representative: Hatzmann, Martin

(57) **Abstract**

Described are methods of treating or preventing an inflammation by administering to a patient in need thereof a pharmacologic dose of glutamine or a glutamine-containing dipeptide in a pharmaceutically acceptable carrier, wherein said dose is selected from a range of 0.09 to 90mg glutamine per kg bodyweight of the patient.

## Description

The invention relates to methods of treating an inflammatory condition, such as heart failure, stroke, systemic inflammatory response syndrome (SIRS) or sepsis, by administering to a patient in need thereof a low dose of glutamine in a pharmaceutically acceptable carrier. Also provided is the use of low dose glutamine for the treatment of an injury relating to ischemia and reperfusion damage and for preventing cardiac and nervous cell damage, and for the manufacture of a medicament for the treatment of such injury.

### Background

L-Glutamine (GLN in three-letter amino acid code, Q in one-letter code), traditionally considered a non-essential amino acid, now appears to be widely used as a conditionally essential nutrient in parenteral feeding during serious injury or illness. Serious illness and injury has shown an increased utilization of GLN by the gut, inflammatory cells, and the kidney. These rapidly replicating cells selectively consume glutamine for its essential role in nucleic acid synthesis and as a preferential fuel during stress.

During good health, GLN is the most abundant amino acid in plasma and skeletal muscle, but circulating and tissue concentrations fall precipitously after injury, surgery, or infection. Specific to Coronary Artery Bypass Grafting (CABG) procedures in human patients, previous research showed that circulating plasma GLN levels fall by at least 30% post CABG. Additional data indicate that myocardial tissue GLN levels also fall significantly post-cardiopulmonary bypass in patients undergoing CABG.

These treatment protocols are based on research that indicates that GLN supplementation may be protective against cellular and organ injury *in vitro* and *in vivo*. Further, it is likely that the fall in plasma GLN following severe illness or injury is detrimental to the function of multiple organs, including the heart. Inflammatory factors play an important role in the production of cellular damage after shock and reperfusion. Glutamine has been used to modulate the inflammatory response. Alanine-glutamine dipeptide (AQ) is a glutamine source. Preliminary studies in human subjects have demonstrated that GLN (provided by peptide sources such as AQ) can improve outcomes in a number of patient populations. Important mechanisms of GLN's protective effects in the myocardium are thought to relate to enhancement of tissue heat shock protein synthesis, support of tissue metabolism, and preservation of tissue redox state.

For example, US Patent Publn. 20050059610, the contents of which are incorporated herein by this reference, describes a treatment of injury or disease utilizing large doses of glutamine. More specifically, the disclosure provides a single large bolus dose treatment of GLN in a pharmaceutically acceptable carrier for either preventing or treating injury or disease by administering to a patient in need. Such a therapeutic approach as disclosed there is suggested for treating acute illness, chronic illness, disease, and injury. Examples of acute illnesses, diseases, and injuries include, but are not limited to, ischemia (both heart and kidney), transplantation, sepsis, hypothermia, lung injury, local and systemic inflammatory diseases, autoimmune conditions, heat shock, heat stroke, and reperfusion injury. This sort of glutamine dosing is said to improve outcomes from cardiac diseases such as coronary artery disease, heart attacks, and cardiopulmonary bypass procedures. Additionally, the therapeutic dosing of GLN is said to be useful in treating other problems including, but not limited to, diseases and injuries associated with tissue metabolism and for increasing heat shock protein or heat shock factor-1 expression *in vivo*. GLN is said to improve outcomes from the problems enclosed above including, but not limited to, ischemia and reperfusion injury in the heart when given as a single dose. For example, a single dose of glutamine dipeptide (alanyl-glutamine) given to a rat 18 hours prior to myocardial injury can improve myocardial function following ischemia and reperfusion injury. This protection is said to be due to preservation of metabolism and glutathione levels, in particular a single large dose of alanyl-glutamine is said to function via preservation of tissue metabolism (ATP content etc.) and preservation of tissue glutathione levels. For example, GLN can be given as a large IV bolus prior to surgery, the parenteral dosage range provided in US 20050059610 is given from 0.1 grams/kg up to 2 grams/kg of glutamine per dose. Preferred in US 20050059610 is alanyl-glutamine as pharmaceutical compound and it was determined that administration following onset of sepsis in the clinically relevant cecal-ligation and puncture model of sepsis in the rat that alanyl-glutamine, given as a single large dose, can restore normal heat shock protein expression to the lung, attenuate inflammatory cytokine release, and improve otherwise defective cellular metabolism in the lung.

Large single or multiple doses of the dipeptide are also used to significantly improve outcomes from severe infections, acute respiratory distress syndrome, and other severe injuries like trauma and severe burns. It has for example been found that high doses of glutamine administered in the form of AQ during resuscitation from hemorrhagic shock will suppress post-shock expression of messenger ribonucleic acid (mRNA) for tumor necrosis factor (TNF-a), interleukin-1 (IL-1β) and inducible nitric oxide synthase (iNOS). The dose can be given upon admission to the emergency room or ICU to prevent morbidity and mortality. Also, the GLN amino acid can improve outcomes from other inflammatory diseases such as inflammatory bowel disease (Crohn's disease, ulcerative colitis), arthritis, multiple sclerosis, and/or lupus.

### THE INVENTION

Described herein is the insight that glutamine dosages administered to critically ill patients are generally too high to treat inflammatory conditions such as SIRS, heart failure or stroke. This is likely caused by the common perception that glutamine is a dietary requirement and should be part of nutritional compositions required for parenteral feeding protocols in, for example, critically ill patients. Here we provide the insight that glutamine at low dosages is perfectly suitable for use as a pharmaceutical composition alone. This observation extends to other inflammatory conditions in patients where glutamine treatment may be therapeutically useful, had not too high dosages been selected because of nutritional bias. It is herein provided that preferred dosages are found at levels considerably below 100 mg glutamine /kg of body weight. A dosage of less than 90 mg/kg, albeit of no exceptional nutritional value, is providing better anti-inflammatory activity than the currently used larger dosages that can be found in the art, especially in acute serious inflammations such as seen with sepsis, SIRS, renal failure, with ischemic-reperfusion injury seen with acute cerebro- or cardiovascular disorders. Glutamine administration in lower dosages as provided herein can also be utilized in inhibiting or treating injury, e.g. skin, epithelial, endothelial or mucosal damage, that can occur as a result of UV radiation, burn injury, aging, chemotherapy, therapeutic radiation or other form of radiation injury.

Preferred dosages herein are found in the ranges from 0.09 to 90 mg/kg of body weight of a (human) subject, more preferred is 0.7 to 90 mg/kg, even more preferred is the range from 0.7 to 70 mg/kg, more preferred is 3 to 70 mg/kg, most preferred range is 7 to 70 mg/kg. These dosages are calculated on the weight of glutamine alone. For example, when a dipeptide is used as source of glutamine, the dosages of dipeptide are higher taking into account the weight of the other amino acid. For example 75 mg/kg alanine-glutamine dipeptide (AQ) correlates to about 52 mg/kg glutamine.

Provided is a method of treating or preventing an acute or chronic inflammation by administering to a patient in need thereof a therapeutically effective dose of glutamine in a pharmaceutically acceptable carrier, wherein the dose is selected from a range of 0.09 to 90 mg glutamine per kg bodyweight of the patient (e.g., a human or animal subject). Furthermore, provided is the use of glutamine or a glutamine-containing dipeptide for the production of a pharmaceutical composition for the treatment or prevention of an inflammatory condition in a subject, wherein the treatment comprises the administration of the glutamine or glutamine dipeptide to the subject at a dose range of 0.09 to 90 mg glutamine per kg of body weight. In one embodiment, said dose range is a daily dose range. In a preferred embodiment, the invention provides the use of glutamine or a glutamine-containing dipeptide for the production of a medicament for the treatment or prevention of an inflammatory condition in a subject, wherein the treatment or prevention comprises the administration of the glutamine or glutamine dipeptide to the subject at a dose range of 0.7 to 70 mg glutamine per kg of body weight. Assessing the bodyweight of such a patient may be done by weighing or by estimating the patient's weight. Final preparation of the pharmaceutical composition comprises calculating the amount of glutamine or glutamine dipeptide needed for the treatment of the patient in question. For preparing the pharmaceutical composition the ultimately required dosages are calculated on the weight of glutamine alone, when for example dipeptides are used, the dosages of dipeptide are higher, for example, 75 mg/kg alanine-glutamine dipeptide (AQ) correlates to about 52 mg/kg glutamine. More preferred ranges are given above. It is even more preferred that said pharmacologic dose or composition of glutamine is administered in the form of a GLN-containing dipeptide, for example wherein the dipeptide is selected from the group consisting essentially of alanyl-glutamine, glycyl-glutamine, leucyl-glutamine, valyl-glutamine, isoleucyl-glutamine, and cysteinyl-glutamine. The dipeptide terminal nitrogen may be acylated, for example wherein the acylating moiety is acetyl.

Also provided is a method of preventing or treating ischemia/reperfusion injury by administering to a patient in need a pharmacologic dose of glutamine in a pharmaceutically acceptable carrier, wherein the dose is selected from a range of 0.09 to 90 mg glutamine per kg bodyweight of the patient. Furthermore, the invention provides use of glutamine or a glutamine dipeptide for the production of a pharmaceutical composition for the prevention or treatment of ischemia/reperfusion injury wherein said glutamine or glutamine dipeptide is used to treat a patient at a range of 0.09 to 90 mg glutamine per kg bodyweight of the patient. Assessing the bodyweight of such a patient may be done by weighing or by estimating the patient's weight. Final preparation of the pharmaceutical composition comprises calculating the amount of glutamine or glutamine dipeptide needed for the patient in question. As said, it is essential for good pharmaceutical activity that the dosages do not exceed 100 mg/kg body weight, and are preferably less high. For preparing said pharmaceutical composition the ultimately required dosages are calculated on the weight of glutamine alone, when, for example, dipeptides are used, the dosages of dipeptide are higher, for example 75 mg/kg alanine-glutamine dipeptide (AQ) correlates to about 52 mg/kg glutamine. More preferred ranges are given above. It is even more preferred that the pharmacologic dose or composition of glutamine is a glutamine dipeptide, for example, wherein the dipeptide is selected from the group consisting essentially of alanyl-glutamine, glycyl-glutamine, leucyl-glutamine, valyl-glutamine, isoleucyl-glutamine, and cysteinyl-glutamine. The dipeptide terminal nitrogen may be acylated, for example wherein the acylating moiety is acetyl.
Furthermore, it is particularly useful that subjects in need of treatment for acute inflammation or ischemia/reperfusion injury can now be treated via a subcutaneous or intra-muscular injection, thereby even allowing self-treatment with an autoinjector or treatment by non-trained or non-medical personnel with said autoinjector. The invention provides an autoinjector containing a sterile injectable solution or dispersion comprising a therapeutically effective dose of glutamine or glutamine-containingdipeptide (such as alanyl-glutamine, glycyl-glutamine, leucyl-glutamine, valyl-glutamine, isoleucyl-glutamine, and cysteinyl-glutamine), or any combination thereof. An autoinjector (or auto-injector) is a medical device designed to deliver a single dose of a particular (typically life-saving) drug. Most autoinjectors are spring-loaded syringes. By design, autoinjectors are easy to use and are intended for self-administration by patients or administration by non-schooled others. The site of injection depends on the drug loaded, but it typically is administered into the thigh or the buttocks. The injectors were initially designed to overcome the hesitation associated with self-administration of needle-based drugs. Such an autoinjector comprising glutamine or glutamine dipeptide can for example be carried along by emergency teams or be on board of ambulances and in first aid centers to provide a quick anti-inflammatory treatment to patients presenting with signs of acute inflammation, injury, trauma, cardiac or cerebral failure. It is preferred to differentiate autoinjectors intended for, respectively, the treatment of adult males, adult females and children. An autoinjector for adult males, on average weighing from 60 - 100 kg, typically contains a sterile injectable solution or dispersion comprising from about 2 to about 5 gram glutamine or equivalent amounts of a glutamine-containing dipeptide (such as alanyl-glutamine, glycyl-glutamine, leucyl-glutamine, valyl-glutamine, isoleucyl-glutamine, and cysteinyl-glutamine), equivalent to a final dose level of 20 - 50 mg/kg glutamine. An autoinjector for adult females on average weighing from 40 - 80 kg typically contains a sterile injectable solution or dispersion comprising a dose of from about 1.6 to about 3.2 gram glutamine or equivalent amounts of glutamine dipeptide (such as alanyl-glutamine, glycyl-glutamine, leucyl-glutamine, valyl-glutamine, isoleucyl-glutamine, and cysteinyl-glutamine), equivalent to a final dose level of approximately 20 - 40 mg/kg glutamine. An autoinjector for children on average weighing from 20 - 40 kg typically contains a sterile injectable solution or dispersion comprising from about 0.8 to about 1.6 gram glutamine or equivalent amounts of glutamine dipeptide (such as alanyl-glutamine, glycyl-glutamine, leucyl-glutamine, valyl-glutamine, isoleucyl-glutamine, and cysteinyl-glutamine), equivalent to a final dose level of 20 - 40 mg/kg glutamine. If only a differentiation between adults and children is required, an autoinjector for adults typically contains a sterile injectable solution or dispersion comprising about 3 gram glutamine or equivalent amounts of glutamine dipeptide (such as alanyl-glutamine, glycyl-glutamine, leucyl-glutamine, valyl-glutamine, isoleucyl-glutamine, and cysteinyl-glutamine). Of course, in areas where general body weight distribution is different, or when one has to calculate in how much of the total content of the autoinjector is generally not administered during the (auto)injection different calculations as to the glutamine or dipeptide content of the autoinjector may be made.

Also provided is a method of protecting organs for transplantation by administering to patient, organ, tissue, or cell in need of such treatment a pharmacologic dose of glutamine in a pharmaceutically acceptable carrier. Also included is the treatment of local and systemic inflammatory diseases and autoimmune conditions and attenuating inflammation or pro-inflammatory cytokine expression in states of health or disease, preventing or treating heat stress, heat stroke, or any other temperature related stress or injury by administering a pharmacologic dose of glutamine in a pharmaceutically acceptable carrier. Further, GLN administered in the low dosages as provided herein can have an anti-inflammatory/anti-cytokine effect and thus can be useful in treating inflammatory illnesses such as, but not limited to, autoimmune disorders such as arthritis (including osteoarthritis and rheumatoid arthritis), Lupus, fibromyalgia, and other related autoimmune diseases, Crohn's disease, irritable bowel syndrome, and other diseases that result in inflammation or increased production of cytokines. GLN administered in dosages as provided herein can be organ-protective. In other words, GLN can be administered to an individual and if organs are removed from the individual, the organs are able to remain viable for an extended period of time outside of the body of the individual. Further, GLN can be administered to an organ, tissue, or cell to provide the organ, tissue, or cell with protection. The benefit of such a property is that if organs can survive a longer period of time after being harvested, then there is a greater likelihood that the organs can be donated to another individual in need of the same. Examples of such organs that can be protected include, but are not limited to, liver, heart, lungs, kidney, and other transplantable organs. Similarly, GLN can be used can be utilized as a portion of a resuscitation fluid or organ transplant preservation solution in combination with other electrolytes. Examples of such fluids/solutions include, but are not limited to, Ringer's lactate solution and other similar solutions known to those of skill in the art. Glutamine as used herein can include any source or form of glutamine known to those of skill in the art. For example, glutamine can be administered as dipeptide, typical glutamine dipeptides such as alanyl-glutamine, glycyl-glutamine, leucyl-glutamine, valyl-glutamine, isoleucyl-glutamine, and cysteinyl-glutamine, or other derivatives thereof are well known to those of skill in the art. Preferably, the GLN is administered in the form of a GLN-containing dipeptide, because the dipeptide can solubilize easily and clears through the body within a few hours, thus eliminating a problem with regard to toxicity. Other dipeptides, such as glycine-valine or methionine-arginine may be added to the pharmaceutical composition comprising glutamine or glutamine dipeptide as provided herein. The dipeptide can also include an acylated terminal nitrogen. Preferably, the acylating moiety for the acylated terminal nitrogen is acetyl.

In the method described herein, the GLN-containing compound of the present invention can be administered in various ways. It should be noted that it can be administered as the compound or as a pharmaceutically acceptable salt, peptide, or chelate and can be administered alone or as an active ingredient in combination with pharmaceutically acceptable carriers, diluents, adjuvants and vehicles. The compounds can be administered subcutaneously or parenterally including intravenous, intra-arterial, intramuscular, intraperitoneally, as well as intrathecal and infusion techniques. Implants of the compounds are also useful. The patient being treated is a warm-blooded animal and, in particular, mammals including man. The pharmaceutically acceptable carriers, diluents, adjuvants and vehicles as well as implant carriers generally refer to inert, non-toxic solid or liquid fillers, diluents or encapsulating material not reacting with the active ingredients of the invention. It is noted that humans are treated generally longer than the mice or other experimental animals exemplified herein which treatment has a length proportional to the length of the disease process and drug effectiveness. The doses may be single doses or multiple doses over a period of several days, but single doses are preferred. The treatment generally has a length proportional to the length of the disease process and drug effectiveness and the patient species being treated. When administering the compound of the present invention parenterally, it will generally be formulated in a unit dosage injectable form (solution, suspension, emulsion). In addition to the dipeptides already mentioned above, it can be contemplated to use dipeptides comprising non-natural amino acids bound to the Gln. These could be chosen to improve solubility, entry into the CNS, muscular availability, and so on.

The pharmaceutical formulations suitable for injection include sterile aqueous solutions or dispersions and sterile powders for reconstitution into sterile injectable solutions or dispersions. The carrier can be a solvent or dispersing medium containing, for example, water, ethanol, polyol (for example, glycerol, propylene glycol, liquid polyethylene glycol, and the like), suitable mixtures thereof, and vegetable oils.

Proper fluidity can be maintained, for example, by the use of a coating such as lecithin, by the maintenance of the required particle size in the case of dispersion and by the use of surfactants. Nonaqueous vehicles such a cottonseed oil, sesame oil, olive oil, soybean oil, corn oil, sunflower oil, or peanut oil and esters, such as isopropyl myristate, may also be used as solvent systems for compound compositions but it is preferred to provide GLN without cottonseed oil, sesame oil, olive oil, soybean oil, corn oil, sunflower oil, or peanut oil and esters, such as isopropyl myristate, that are commonly used in parenteral nutrition, when glutamine activity is desired for its pharmaceutical effect alone. Additionally, various additives which enhance the stability, sterility, and isotonicity of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin. According to the present invention, however, any vehicle, diluent, or additive used would have to be compatible with the compounds. Sterile injectable solutions can be prepared by incorporating the compounds utilized in practicing the present invention in the required amount of the appropriate solvent with various of the other ingredients, as desired. A pharmacological formulation of the present invention can be administered to the patient in an injectable formulation containing any compatible carrier, such as various vehicle, adjuvants, additives, and diluents; or the compounds utilized in the present invention can be administered parenterally to the patient in the form of slow-release subcutaneous implants or targeted delivery systems such as monoclonal antibodies, vectored delivery, iontophoretic, polymer matrices, liposomes, and microspheres. Examples of patents disclosing delivery systems useful in the present invention include: U.S. Pat. Nos. 5,225,182; 5,169,383; 5,167,616; 4,959,217; 4,925,678; 4,487,603; 4,486,194; 4,447,233; 4,447,224; 4,439,196; and 4,475,196, the contents of the entirety of each of which are incorporated herein by this reference. Many other such implants, delivery systems, and modules are well known to those skilled in the art.

### EXAMPLES

### Example 1

### Mice renal failure test.

Dipeptides are tested and compared with PBS (control) in a double blind animal study for a dipeptide's relative ability to aid recovery in a mouse renal ischemia reperfusion or mouse renal failure test. Under inhalation anesthesia, the left kidney with its artery and vein is isolated and occluded for 25 minutes using a microvascular clamp. During surgery, animals are placed on a heating path to maintain body temperature at 37°C. Five minutes before placing the clamp, and 5 minutes before releasing the clamp, dipeptide, dissolved in 0.1 mL of sterile saline, is administered intravenously. After reperfusion of the left kidney the right kidney is removed. Kidney function is assessed by measuring blood urea nitrogen before clamping, and at 2, 24, and 72 hours after reperfusion, by determining mRNA levels for cytokine expression, and such.

In short, in this test, the mice are anesthetized, and one kidney from each mouse is removed. The other kidney is tied off for 25 minutes, and the ischemia levels in the kidney are allowed to increase. Both before and/or after tying off, GLN-containing dipeptide is administered to 8 to 10 different mice per group (dosages vary per group from for example 0.7, 3, 7, 30 and 70 mg dipeptide / kg body mass intravenously). The mortality of the mice is determined for each oligopeptide as well as for example the BUN or creatinine concentration at two hours, 24 hours and 72 hours. Two dipeptides AQ and LQ are for example tested in a dose-response manner in the mice renal failure test. Dipeptides are tested at dosages of 0.7, 3, 7, 30 and 70 mg/kg body weight given intravenously. Typical results are shown in Table 1, wherein a score of for instance 0-9 indicates that none of the 9 test animals within that group has died. Likewise, a score of 6-8 indicates that six out of the 8 mice have died.

| Table 1 Mortality in mice renal failure experiment | **24h** | **72h** |
|---|---|---|
| PBS | 0-9 | 6-8 |
| AQ 0.7 mg/kg | 0-9 | 2-9 |
| AQ 3.0 mg/kg | 0-10 | 1-9 |
| AQ 7.0 mg/kg | 0-10 | 0-9 |
| AQ 30.0 mg/kg | 0-8 | 1-10 |
| AQ 70.0 mg/kg | 0-9 | 1-9 |
| LQ 0.7 mg/kg | 0-9 | 2-9 |
| LQ 3.0 mg/kg | 0-10 | 1-9 |
| LQ 7.0 mg/kg | 1-10 | 1-10 |
| LQ 30.0 mg/kg | 0-10 | 0-9 |
| LQ 70.0 mg/kg | 0-8 | 3-9 |

### Example 2

Rat hemorrhagic shock study.

Inflammatory factors play an important role in the production of cellular damage after shock and reperfusion. Glutamine has been used to modulate the inflammatory response. Alanine-glutamine dipeptide (AQ) is a glutamine source. Without wishing to be bound by any theory, the present inventors hypothesize that AQ given during resuscitation will suppress post-shock expression of messenger ribonucleic acid (mRNA) for tumor necrosis factor (TNF-alpha).

Methods: Rats are randomly assigned to groups. Under isoflurane anesthesia, the femoral artery and vein are cannulated. Hemorrhagic shock is induced by withdrawing blood through the arterial cannula until the mean arterial pressure (MAP) is 25-30 mm Hg and maintained at the level for 30 minutes with further withdrawals. Resuscitation is carried out by giving 21 mL/kg Ringer's lactate (LR) with or without the administration of AQ (intravenously at ranges from 0.7 to 70 mg/kg) and returning the shed blood. Controls are normal (anesthesia only), sham (surgical preparation), and shock (preparation and shock). Rats (n = 80, 10 per group) are killed 30 minutes after completion of resuscitation. Liver samples are collected, and total RNA is isolated for reverse transcription-polymerase chain reaction analysis of mRNA (TNF-a). Results: MAP recovers more quickly in the AQ groups than in the LR group. Increased expression of liver mRNA for TNF-alpha is seen after hemorrhagic shock and resuscitation. AQ treatment at the low dosages tested reduces mRNA expression for TNF-alpha.

## Claims

1. A method of treating or preventing an inflammatory condition by administering to a subject in need thereof a therapeutically effective dose of glutamine or a pharmaceutically acceptable salt thereof in a pharmaceutically acceptable carrier, wherein said dose is within a range of from 0.09 to 90 mg glutamine per kg bodyweight of the subject.

2. The method according to claim 1, wherein said dose ranges from 0.7 to 70 mg / kg bodyweight.

3. The method according to claim 1 or claim 2, wherein said therapeutically effective dose of glutamine is administered in the form of a glutamine-containing dipeptide or pharmaceutically acceptable salt thereof.

4. The method according to claim 3, wherein said dipeptide is selected from the group consisting of alanyl-glutamine, glycyl-glutamine, leucyl-glutamine, valyl-glutamine, isoleucyl-glutamine, and cysteinyl-glutamine.

5. Use of glutamine or a glutamine-containing dipeptide, or a pharmaceutically acceptable salt thereof, for the production of a medicament for the treatment or prevention of an inflammatory condition in a subject, wherein said treatment comprises administering glutamine or a glutamine dipeptide in a dose range of 0.09 to 90 mg glutamine per kg bodyweight of the subject.

6. A method of preventing or treating ischemia/reperfusion injury by administering to a patient in need thereof a therapeutically effective dose of glutamine, or a pharmaceutically acceptable salt thereof, in a pharmaceutically acceptable carrier, wherein said dose is selected from a range of 0.09 to 90 mg glutamine per kg bodyweight of the patient.

7. The method according to claim 6, wherein said dose range is from 0.7 to 70 mg.

8. The method according to claim 6 or 7, wherein said glutamine is administered in the form of a glutamine-containing dipeptide.

9. Use of glutamine or a glutamine-containing dipeptide or a pharmaceutically acceptable salt thereof for the production of a pharmaceutical composition for the prevention or treatment of ischemia/reperfusion injury wherein said glutamine or glutamine dipeptide is used to treat a patient at a range of 0.09 to 90 mg glutamine per kg bodyweight of the patient.

10. An autoinjector containing a sterile injectable solution or dispersion comprising glutamine or glutamine-containing dipeptide, or a pharmaceutically acceptable salt thereof.

11. Autoinjector according to claim 10, comprising a dipeptide selected from the group consisting of alanyl-glutamine, glycyl-glutamine, leucyl-glutamine, valyl-glutamine, isoleucyl-glutamine, and cysteinyl-glutamine.
